# EUROPEAN PATENT SPECIFICATION

(11) **EP 2 453 892 B1**
(45) Date of publication and mention of the grant of the patent: **29.05.2013**
(21) Application number: 09787298.0
(22) Date of filing: 16.07.2009
(51) Int. Cl.: A61K 31/4196, A61K 31/4402, A61K 31/59, A61K 31/593, A61K 47/48, A61P 35/00

(54) **PHARMACEUTICAL COMPOSITIONS AND THEIR USE FOR TREATING CANCER**
PHARMAZEUTISCHE ZUSAMMENSETZUNGEN UND DEREN VERWENDUNG ZUR KREBSTHERAPIE
COMPOSITIONS PHARMACEUTIQUES ET LEUR UTILISATION DANS LE TRAITEMENT DU CANCER

(43) Date of publication of application: 23.05.2012
(73) Proprietor: Institut National de la Santé et de la Recherche Médicale, 75013 Paris (FR); Centre National de la Recherche Scientifique CNRS, 75016 Paris (FR)
(72) Inventor: CRUZ MOURA, Ivan, F-75870 Paris Cedex 18 (FR); HERMINE, Olivier., F -75015 Paris (FR); COULON, Séverine., F -75015 Paris (FR); CALLENS, Celine., F-75015 Paris (FR)
(74) Representative: Domenego, Bertrand
(86) International application number: PCT/IB2009/054210
(87) International publication number: WO 2011/007208

(56) References cited:
- WO-A1-2005/111082
- VERBEELEN D ET AL: "Vitamin D, desferrioxamine and aluminum-induced bone disease in uremic rats" NEPHRON, S. KARGER AG, SWITZERLAND, vol. 53, no. 1, 1 January 1989 (1989-01-01), pages 54-60, XP009130660 ISSN: 0028-2766
- GONZÁLEZ SUÁREZ I ET AL: "Efecto de la desferrioxamina y de la deferiprona sobre la secreción de osteocalcina en células tipo osteoblasto = Effect of desferrioxamine and deferiprone on osteocalcin secretion in osteoblast-type cells" NEFROLOGÍA, AULA MÉDICA, MADRID, ES, vol. 23, no. suppl. 2, 1 January 2003 (2003-01-01), pages 27-31, XP009130657 ISSN: 0211-6995
- DE SANCTIS V ET AL: "Reversible heart failure in a thalassaemic patient secondary to hypocalcemia and hypoparathyroidism" RIVISTA ITALIANA DI MEDICINA DELL'ADOLESCENZA, SCRIPTA MANENT EDIZIONI, IT, vol. 6, no. 2, 1 May 2008 (2008-05-01), pages 39-41, XP009130728
- BEER TOMASZ M ET AL: "Double-blinded randomized study of high-dose calcitriol plus docetaxel compared with placebo plus docetaxel in androgen-independent prostate cancer: a report from the ASCENT Investigators." 20 February 2007 (2007-02-20), JOURNAL OF CLINICAL ONCOLOGY : OFFICIAL JOURNAL OF THE AMERICAN SOCIETY OF CLINICAL ONCOLOGY 20 FEB 2007, VOL. 25, NR. 6, PAGE(S) 669 - 674 , XP002572607 ISSN: 1527-7755 cited in the application paragraph ["Introduction"]
- WHITNALL MEGAN ET AL: "A class of iron chelators with a wide spectrum of potent antitumor activity that overcomes resistance to chemotherapeutics." 3 October 2006 (2006-10-03), PROCEEDINGS OF THE NATIONAL ACADEMY OF SCIENCES OF THE UNITED STATES OF AMERICA 3 OCT 2006, VOL. 103, NR. 40, PAGE(S) 14901 - 14906 , XP002572608 ISSN: 0027-8424 cited in the application abstract
- MA Y ET AL: "Identification and characterization of noncalcemic, tissue-selective, nondecosteroidal vitamin D receptor modulators", JOURNAL OF CLINICAL INVESTIGATION, AMERICAN SOCIETY FOR CLINICAL INVESTIGATION, US, vol. 116, no. 4, 3 April 2006 (2006-04-03) , pages 892-904, XP003020909, ISSN: 0021-9738, DOI: 10.1172/JCI25901
- POLEK T C ET AL: "NOVEL NONSECOSTEROIDAL VITAMIN D RECEPTOR MODULATOR INHIBITS THE GROWTH OF LNCAP XENOGRAFT TUMORS IN ATHYMIC MICE WITHOUT INCREASED SERUM CALCIUM", PROSTATE, WILEY-LISS, NEW YORK, NY, US, vol. 49, 1 January 2001 (2001-01-01), pages 224-233, XP008056107, ISSN: 0270-4137, DOI: 10.1002/PROS.1138

## Description

The present invention relates to compounds and pharmaceutical compositions suitable for use in the treatment or prevention of cancer, in particular for use in the treatment or prevention of acute myeloid leukemia (AML).

### Background of the invention

Acute myeloid leukemia (AML) is a heterogeneous malignant disorder originating from mutations in progenitor cells which cause an unrestrained proliferation of undifferentiated myeloid blasts. AML is the most common acute leukemia affecting adults and its incidence increases with age. Although AML is a relatively rare disease, accounting for approximately 1.2% of cancer deaths in the United States (Jemal et al. (2002) CA Cancer J Clin 52(1):23-47), its incidence is expected to increase as the population ages.

In AML patients, bone marrow is usually highly infiltrated by blast cells, resulting in a drop in red blood cells, platelets and normal white blood cells. The symptoms of AML include fatigue, shortness of breath, easy bruising and bleeding, and increased risk of infection. Although several risk factors for AML have been identified, the specific cause of AML remains unclear. AML progresses rapidly and is typically fatal within weeks or months if left untreated. AML leukemogenesis occurs as multistep events (Gilliland et al. (2004) Hematology Am Soc Hematol Educ Program 80-97). These events are classified into two groups: The first group involves gene alterations which confer proliferative and/or survival advantage to hematopoietic progenitors (*e.g.* RAS, FLT3 or c-KIT mutations). The second involves alterations in transcription factors (or transcription co-activators), most frequently induced by chromosomal translocations but also point mutations (*e.g.* CEBPα, NPM1), which affect hematopoietic differentiation.

Treatment of AML consists primarily of conventional chemotherapy and, if the majority of patients achieve remission after initial therapeutic induction most of them,relapse. Post-remission treatments, designed to ensure long-term disease-free survival include high-dose chemotherapy with autologous stem cell rescue, or allogeneic bone marrow transplantation (BMT). However elderly patients, who constitute the majority of AML patients, are not eligible for BMT. Despite aggressive therapy, only 20%-30% of patients enjoy long-term disease-free survival. Thus, other therapeutics are needed.

Iron is required as a cofactor for a number of critical cellular enzymes involved in energy metabolism and cell proliferation and thus is essential for all living cells. Different studies have shown that tumor cells strongly express the transferrin receptor (TfR1/CD71) and are more sensitive to iron deprivation than non-tumor cells (Faulk et al. (1980) Lancet 2(1):390-92).

The inventors previously characterized a monoclonal antibody (A24) which binds strongly to TfR1 when this receptor is highly expressed on the cell surface. A24 binding induces internalization of the receptors in lysosomal compartments where they are degraded and also cell apoptosis by iron deprivation (Moura et al. (2004) Blood 103(5):1838-45, Lepelletier et al. (2007) Cancer Res 67(3):1145-54). A24 was shown to reduce specifically the proliferation of tumor cells and not of the normal cells. Some studies have shown the efficacy of A24 to eradicate leukemia/lymphoma cells in adult T-cell leukemia/lymphoma (ATL) and in Mantle cell lymphoma (MCL) (Moura et al. (2004) Blood 103(5):1838-45, Lepelletier et al. (2007) Cancer Res 67(3):1145-54).

Others studies have recently demonstrated that iron chelators also possess anti-proliferative properties *in vitro* and *in vivo*. The efficacy of different iron chelators has been shown on human xenografts in nude mice (Whitnall et al. (2006) Proc Natl Acad Sci USA 103(40):14901-6).

The anti-proliferative, differentiating and pro-apoptotic effects of vitamin D3 (VD) have been shown on normal or pathological epithelial tissues. VD belongs to the steroid superfamily. VD associates with its cognate receptor (VDR) to form a heterodimer with the retinoid X receptor (RXR). This complex then binds to the VD responsive element (VDRE), thereby inducing changes in gene transcription. VD also activates the MAP kinase (MAPK) pathways to modulate the transcription of genes implicated in cell differentiation.

*In vitro* VD promotes the expression of genes associated to originally differentiated cells, in cell lineage derived from head, neck, prostate or colon cancer (Akutsu et al (2001) Mol Endocrinol 15(7) :1127-39, Palmer et al. (2003) Cancer Res 63(22) :7799-806).

*In vitro*, calcitriol (the active form of vitamin D3) exhibits antiproliferative activity in squamous cell carcinoma and prostatic adenocarcinoma and enhances the antitumor activity of the classical chemotherapeutic agents (Hershberger et al. (2001) Clin Cancer Res 7(4):1043-51).

In a prospective randomized study comparing the use of a wildely used chemotherapeutic agent (docetaxel) associated or not with of high-dose calcitriol in prostate cancer, the patients survival was found to be increased in the arm VD/Doctaxel (Beer et al. (2007) J Clin Oncol 25(6):669-74). However the response to vitamin D3 declines quickly after repeated treatment and thus its effect is limited in time.

Finally, some authors have shown *in vitro* the efficiency of vitamin D3 as a differentiating agent and its use in AML treatment has been proposed (Makashima et al. (1998) Br J Cancer 77(1):33-9). The association of chemotherapy (aracytine or 6-thioguanine) and differentiating agents (vitamin D and retinoic acid) was evaluated once on 26 patients allowing 50% of remissions (27% complete and 23% partial) (Ferrero et al. (2004) Haematologica 89(5):619-20).

To conclude, iron deprivation and VD have been tested as anti-tumoral therapy but none of the above agents have proven liable to completely treat individuals afflicted with cancer, such AML. Thus, there is a need of new therapeutic alternatives which could provide new perspectives in particular in AML treatment.

### Summary of the invention

The present invention arises from the unexpected finding by the inventors that iron chelating agents act synergistically with vitamin D3, in particular through the activation of mitogen-activated protein kinase (MAPK) pathway. This association was proved to be effective *in vitro* in AML blasts and *in vivo* in AML xenografted tumors in mice. The inventors have also shown that the associated therapy also improved the state of an AML patient refractory to chemotherapy and induced cell differentiation of patient's blasts.

Thus, the present invention relates to a pharmaceutical composition comprising:
a) at least one iron uptake inhibitor as described in claim 1,
b) at least one compound able to induce the activation of MAPK pathways, which is at least one vitamin D and/or at least one analog thereof and/or at least one vitamin D receptor modulator,
and optionally a pharmaceutically acceptable carrier, in particular for use in the prevention or treatment of cancer.

The present invention also relates to products containing:
a) at least one iron uptake inhibitor as defined in claim 1
b) at least one compound able to induce the activation of MAPK pathways, which is at least one vitamin D and/or at least one analog thereof and/or at least one vitamin D receptor modulator,
as a combined preparation for simultaneous, separate or sequential use in the prevention or treatment of cancer.

The present invention also relates to the use of at least one iron uptake inhibitor and at least one compound able to induce the activation of MAPK pathways, which is at least one vitamin D and/or at least one analog thereof and/or at least one vitamin D receptor modulator for the manufacture of a medicament intended for the prevention or the treatment of cancer.

The present patent also describes a method for the prevention and/or the treatment of cancer in an individual, comprising administering the individual with a prophylactically or therapeutically effective quantity of at least one iron uptake inhibitor and at least one compound able to induce the activation of MAPK pathways, in particular at least one vitamin D and/or at least one analog thereof and/or at least one vitamin D receptor modulator (VDRMs).

### Detailed description of the invention

As defined herein the expression "iron uptake inhibitor" refers to a compound which is active to reduce or to prevent iron uptake by cells.

Preferably, the iron uptake inhibitor is selected from the group consisting of an iron chelator and a transferrin receptor inhibitor.

As used herein, the expressions "iron chelator" or "iron chelating compound" are used indifferently and relate to a compound that binds iron. An iron chelating compound bound or complexed with iron is referred to herein as an iron chelate. Preferably, the iron chelating compound may be a bidentate, a tridentate, a tetradentate or a higher multidentate compound. A bidentate, tridentate, tetradentate or higher multidentate iron chelating compound refers to compounds which bind iron using two, three, four or more separate binding sites, respectively. Iron chelating compounds of the invention include chelation compounds that can bind to all oxidation states of iron including, for example, iron (-II) state, iron (-I) state, iron (0) state, iron (I) state, iron (II) state (ferrous), iron (III) state (ferric), iron (IV) state (ferryl) and/or iron (V).

Examples of specific bidentate iron chelators comprise 1,2-dimethyl-3-hydroxypyridin-4-one (Deferiprone, DFP or Ferriprox) and 2-deoxy-2-(N-carbamoylmethyl-[N'-2'-methyl-3'-hydroxypyridin-4'-one])-D-glucopyranose (Feralex-G). Examples of specific tridentate iron chelators comprise pyridoxal isonicotinyl hydrazone (PIH), 4,5- dihydro-2-(2,4-dihydroxyphenyl)-4-methylthiazole-4-carboxylic acid (GT56-252), 4,5- dihydro-2-(3'-hydroxypyridin-2'-yl)-4-methylthiazole-4-carboxylic acid (desferrithiocin or DFT) and 4-[3,5-bis(2-hydroxyphenyl)-[1,2,4]triazol-1-yl]benzoic acid (ICL-670, Deferasirox). The iron chelator can also be substituted 3,5-diphenyl-1,2,4-triazoles in the free acid form, salts thereof and its crystalline forms (as described in WO 97/49395). Examples of specific hexadentate iron chelators comprise N,N'-bis(o-hydroxybenzyl) ethylenediamine-N,N'-diaceticacid (HBED), N-(5-C3-L(5 aminopentyl) hydroxycarbamoyl)-propionamido)pentyl)-3(5-(N-hydroxyacetoamido)-pentyl) carbamoyl)-proprionhydroxamic acid (deferoxamine, desferrioxamine or DFO) and hydroxymethyl-starch-bound deferoxamine (S-DFO). Further derivatives of DFO include aliphatic, aromatic, succinic, and methylsulphonic analogs of DFO and specifically, sulfonamide-deferoxamine, acetamide- deferoxamine, propylamide deferoxamine, butylamide-deferoxamine, benzoylamide-deferoxamine, succinamide-derferoxamine, and methylsulfonamide-deferoxamine.

An iron chelator according to the invention can also be a siderophore or a xenosiderophore. Examples of siderophore or xenosiderophore comprise hydroxamates and polycarboxylates. The hydroxamates contain an N-[delta]-hydroxyornithine moiety and are generally categorized into four exemplary families. One category includes rhodotorulic acid, which is the diketopiperazine of N-[delta]-acetyl-L-N [delta]-hydroxyornithine. Included within this category are derivatives such as dihydroxamate named dimerum acid. A second category includes the coprogens, which contain an N-[delta]-acyl-N-[delta]-hydroxy-L-ornithine moiety. Coprogens also can be considered trihydroxamate derivatives of rhodotorulic acid with a linear structure. A third category includes the ferrichromes, which consist of cyclic peptides containing a tripeptide of N-[delta]-acyl-N-[delta]-hydroxyornithine and combinations of glycine, serine or alanine. The fourth exemplary category includes the fusarinines, also called fusigens, which can be either linear or cyclic hydroxamates. Fusarinine is a compound characterized by N acylation of N-hydroxyornithine by anhydromevalonic acid. The polycarboxylates consist of a citric acid-containing polycarboxylate called rhizoferrin. The molecule contains two citric acid units linked to diaminobutane. Other categories of siderophores useful as iron chelators in the compositions of the invention include, for example, the phenolate-catecholate class of siderophores, hemin, and [beta]-ketoaldehyde phytotoxins.

The iron chelator according to the invention can also be a thiosemicarbazone, Triapine (3-aminopyridine-2-carboxaldehyde thiosemicarbazone, 3AP) which is a synthetic heterocyclic carboxaldehyde thiosemicarbazone with potential antineoplastic activity, pyridoxal isonicotinoyl hydrazone analogs like di-2-pyridylketone thiosemicarbazone (DpT), di-2-pyridylketone isonicotinoyl hydrazone (PKIH) analogs and di-2-pyridylketone-4,4,-dimethyl-3-thiosemicarbazone (Dp44mT).

Preferably the iron chelator of the invention is selected from the group consisting of deferoxamine, deferasirox, deferiprone, Triaprine and Dp44mT.

The expression "transferrin receptor inhibitor" refers to any compound which binds to the transferrin receptor and inhibits or prevents fixation of transferrin or inhibits or prevents the internalisation of the transferrin receptor/transferrin complex into cells.

Transferrin is the main blood plasma protein involved in the transport of iron. Iron loaded transferrin (Fe-Tf) binds to its cellular receptor (TfR) and the complex Fe-Tf/TfR is internalized in a vesicle. The pH of the vesicle is acidified wich induces the release of iron by transferrin. The receptor is then recycled to the cell surface (Irie et al. (1987) Am J Med Sci 293:103-11).

The transferrin receptor inhibitor according to the invention can for example be selected from the group consisting of an anti-transferrin receptor antibody, synthetic or native sequence peptides and small molecule antagonists and aptamers which bind to the transferrin receptor.

Preferably, the transferrin receptor inhibitor according to the invention is an anti-transferrin receptor antibody, more preferably an monoclonal antibody. More preferably, this antibody is A24 (deposited at the CNCM (Collection nationale de Cultures de Microorganismes, 25 rue du Docteur Roux, Paris) on May 10, 2001, under number I-2665) notably described in WO 2005/111082 (Moura et al. (2001) J Exp Med 194(4):417-25), Moura et al. (2004) Blood 103(5):1838-45, Lepelletier et al. (2007) Cancer Res 67(3):1145-54).

As used herein the term "antibody" is used in the broadest sense and specifically covers intact monoclonal antibodies, polyclonal antibodies, chimeric, humanized or human antibodies, diabodies, multispecific antibodies (*e.g.* bispecific antibodies) formed from at least two intact antibodies, and also antibody fragments. Examples of antibody fragments include Fv, Fab, F(ab')₂, Fab', dsFv, scFv, sc(Fv)₂.

MAPK pathway is related to any biological pathway which implicates Mitogen-activated protein kinases (MAPK) which are serine/threonine-specific protein kinases that respond to extracellular stimuli and regulate various cellular activities, such as gene expression, mitosis, differentiation, and cell survival/apoptosis. MAPK pathways are well known from the person skilled in the art and are notably described in Brown et al. ((2008) Handb Exp Pharmacol 1186:205-35) or Raman et al. ((2007) Oncogene 26(22):3100-12). Example of MAPK can be Jun Kinase, p38 and extracellular signal regulated kinases. As intended herein MAPK pathway agonist are able to induce MAP kinase pathways. Example of MAPK pathway agonist according to the invention can be iron chelator and vitamine D, analog thereof and vitamin D receptor modulator.

As used herein the term "vitamin D" comprises all the forms of vitamin D, as for example vitamin D1, vitamin D2 (ergocalciferol), vitamin D3 (cholecalciferol), vitamin D4 (22-dihydroergocalciferol) and vitamin D5 (sitocalciferol). Preferably, the "at least one vitamin D" according to the invention is vitamin D3, more preferably vitamin D3 in its active form 1α,25-dihydroxycholecalciferol D3 (1,25-(OH)₂D3 or calcitriol).

As intended herein a "vitamin D analog" or a "vitamin D receptor modulator" are able to bind to the vitamin D receptor (VDR) and preferably are able to induce cell differentiation upon binding.

Tests for determining the capacity of a vitamin D analog or of a vitamin D receptor modulator to bind to the vitamin D receptor are well known to the person skilled in the art. Preferably, this capacity can be evaluated by estimating the specific binding of the analog or of the vitamin D receptor modulator on a cell extract. For example, in a typical binding experiment, soluble cell extract obtained by sonication is incubated with increasing concentration of vitamin D analog or of vitamin D receptor modulator. Bounds and free analogs can be separated by the hydroxylapatite method. Specific binding can be calculated by subtracting nonspecific binding obtained in the presence of an excess 1,25-(OH)₂D3 from the total binding measured in absence of 1,25-(OH)₂D3 (Skowronski et al. (1995) Endocrynology 136(1): 20-26).

The capacity of a vitamin D analog or of a vitamin D receptor modulator to induce cell differentiation can be measured by various methods well known to the person skilled in the art. For example, this capacity can be estimated by the measure of the induction of PSA (a marker of cellular differentiation) in a LNCaP cell line after incubation with the analog or with the vitamin D receptor modulator as described in Skowronski *et al.* (op. cit.).

Many vitamin D analogs are well known in the state of the art. The expression "vitamin D analog" notably encompasses vitamin D metabolites, vitamin D derivatives and vitamin D precursors, preferably it encompasses vitamin D3 metabolites, vitamin D3 derivatives and vitamin D3 precursors.

Vitamin D analogues according to the invention can retain the secosteroid structure with modified side chain structures around the C-24 position. For example, vitamin D analogues can be paricalcitol (19-nor-1α(OH)2D2), ILX23-7553 (16-ene-23-yne-1 α,25(OH)2D3), OCT (Maxacalcitol, 22-oxa-1α,25(OH)2D3) and EB1089 (Seocalcitol, 1 α-dihydroxy-22,24-diene-24,26,27-trihomo-vitamin D3) or cholecalciferol.

More preferably, the vitamin D analog according to the invention is selected from the group consisting paricalcitol, OCT, EB1089 and cholecalciferol. More preferably the vitamin D analog is cholocalciferol.

Vitamin D receptor modulators (VDRMs) according to the invention are preferably non-secosteroidal compounds that have been shown to be less hypocalcemic than the VD analogues, as for example the compounds mentioned in US 2008/0200552. More preferably, the at least one vitamin D receptor modulator (VDRMs) is selected from the group consisting of LY2108491, LY2109866 and LG190119 (Ma et al. (2006) J Clin Invest 116(4):892-904, Polek et al (2001) Prostate 49(3):224-33)

The expression "pharmaceutically acceptable carrier" refers to molecular entities and compositions that do not produce an adverse, allergic or other untoward reaction when administered to a mammal, especially a human, as appropriate. A pharmaceutically acceptable carrier or excipient refers to a nontoxic solid, semi-solid or liquid filler, diluent, encapsulating material or formulation auxiliary of any type.

The form of the pharmaceutical compositions, the route of administration, the dosage and the regimen according to the invention depend upon the condition to be treated, the severity of the illness, the age, weight, and sex of the patient, etc.

The pharmaceutical compositions of the invention can notably be formulated for an oral (preferably for administration of VD and iron chelators), intravenous (preferably for administration of VD, transferrin receptor inhibitor and iron chelators), subcutaneous (preferably for the administration of iron chelators) or intramuscular (preferably for the administration of VD) administration.

Preferably, the pharmaceutical composition according to the invention comprises vitamin D3 and monoclonal antibody A24. Also preferably, the pharmaceutical composition according to the invention comprises vitamin D3 and deferoxamine and/or deferasirox.

By way of example, the at least one iron uptake inhibitor according to the invention may be administered at a concentration of about 0.1 to 1000 mg/kg body weight/day or preferably about 1 to 100 mg/kg body weight/day. Preferably, deferasirox may be administered at a concentration of about 1 to 50 mg/kg body weight/day and more preferably at a concentration of about 10-30 mg/kg body weight/day. Preferably, deferoxamine may be administered at a concentration of about 10 to 100 mg/kg body weight/day and more preferably at a concentration of about 60-80 mg/kg body weight/day.

By way of example, the at least one vitamin D and/or at least one analog thereof and/or at least one vitamin D receptor modulator (VDRMs) according to the invention may be administered at a concentration of about 0.001 to 100 g/kg body weight/day or about 0.01 to 50 g/kg body weight/day or about 0.1 to 5 g/kg/ body weight/day more preferably about 0.5 g/kg body weight/day.

The at least one iron uptake inhibitor can be administered prior to, concomitantly with, or subsequent to the administration of the at least one vitamin D and/or at least one analog thereof and/or at least one vitamin D receptor modulator (VDRM) to an individual which had, has, or is susceptible to developing a cancer as defined above. The at least one iron uptake inhibitor and the at least one vitamin D and/or at least one analog thereof and/or at least one vitamin D receptor modulator (VDRM) can be administered to an individual in a sequence and within a time interval such that the first binding molecule can act together with the second binding molecule to provide an increased benefit than if they were administered otherwise. Preferably, the at least one iron uptake inhibitor and the at least one vitamin D and/or at least one analog thereof and/or at least one vitamin D receptor modulator (VDRM) are administered together.

The pharmaceutical composition according to the invention can be use in the prevention or treatment of cancer. Preferably, the cancer is a hematopoietic malignancy.

In the context of the invention, the term "treatment or prevention" means reversing, alleviating, inhibiting the progress of, or preventing the disorder or condition to which such term applies, or one or more symptoms of such disorder or condition. In particular, the treatment of the disorder may consist in reducing the number of malignant cells. Most preferably, such treatment leads to the complete depletion of malignant cells.

Preferably, the individual to be treated is a human or non-human mammal (such as a rodent (mouse, rat), a feline, a canine, or a primate) affected or likely to be affected with cancer. Preferably, the individual is a human.

The terms "cancer" and "malignancy" refer to or describe the pathological condition in mammals that is typically characterized by unregulated cell growth. More precisely, in the use of the invention, diseases, namely tumors that express Vitamin D receptor are most likely to respond to Vitamin D modulators. Furthermore, inventors make hypothesis that iron deprivation by using iron uptake inhibitor restores sensibility in the vitamin D by surexpression of its receptor in the cell surface. In particular, the cancer may be associated with a solid tumor or unregulated growth of undifferentiated hematopoietic bone marrow cells (hematopoietic stem cell). Examples of cancers that are associated with solid tumor formation include breast cancer, bladder cancer, uterine/cervical cancer, oesophageal cancer, pancreatic cancer, colon cancer, colorectal cancer, kidney cancer, ovarian cancer, prostate cancer, head and neck cancer, non-small cell lung cancer and stomach cancer.

Preferably, the cancer or malignancy according to the invention is due to an unregulated growth of undifferentiated hematopoietic bone marrow cells (hematopoietic stem cell).

As intended herein the expression "hematopoietic stem cell (HSC)" refers to adult multipotent stem cells that give rise to all the blood cell types including for example myeloid lineages (monocytes and macrophages, neutrophils, basophils, eosinophils), erythrocytes, megakaryocytes/platelets, and lymphoid lineages (T-cells, B-cells, NK-cells).

The expression "hematopoietic stem cell malignancy" or "hematopoietic malignancy" according to the invention comprises acute myeloid leukemia (AML), acute lymphoblastic leukemia, chronic myeloid, lymphoid leukemia, lymphoma and myelodysplastic syndrome (as defined in 2001 WHO classification). Preferably, the cancer according to the invention is selected from the group consisting of myelodysplastic syndrome and acute myeloid leukaemia.

### Brief description of the drawings

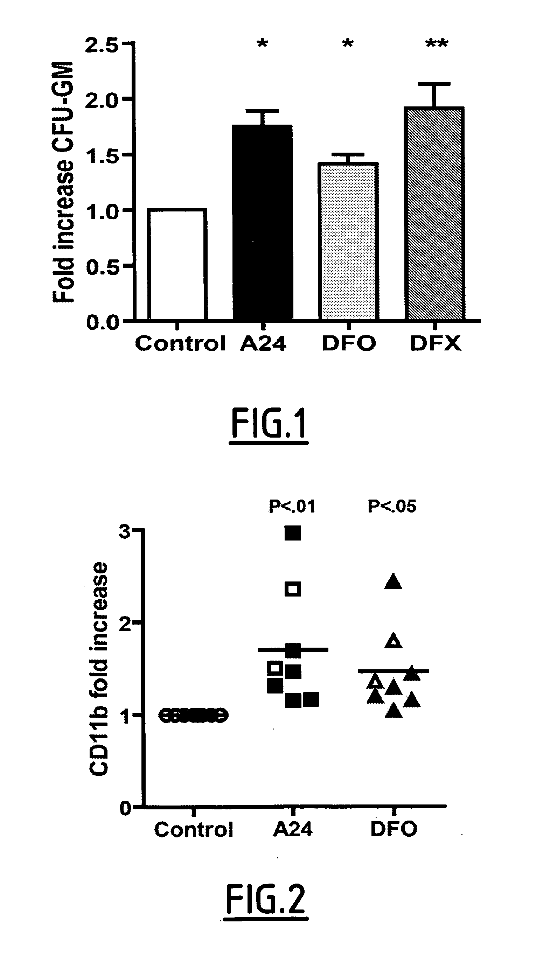

**FIG.1** depicts the colony-forming unit macrophage (CFU-GM) ratio obtained from semi-solid cultures of CD34⁺ cord blood cells treated by A24 (A24), deferoxamine (DFO) or deferasirox (DFX) compared to untreated cells (Control).
**FIG. 2** depicts the expression of CD11b in AML patients' blasts treated with A24 and DFO for 72h. The values obtained are normalized by calculating the increase of the MFI relative to control cells. AML subtypes are distinguished: filled symbols represent MO/M1/M2 AML subtypes whereas empty symbols represent M4/M5 subtypes. Mean values obtained from eight patients are represented.

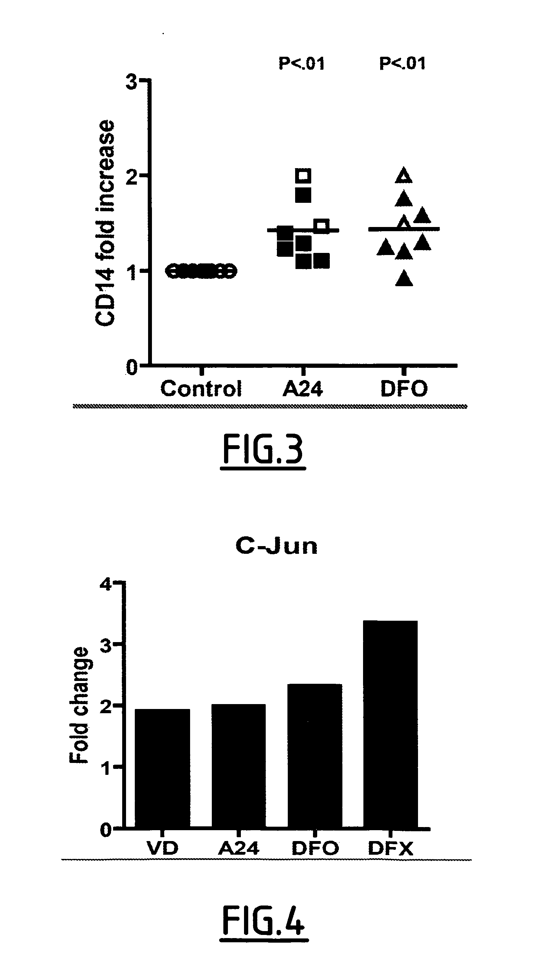

**FIG. 3** depicts the expression of CD14 in AML patients' blasts treated with A24 and DFO for 72h. The values obtained are normalized by calculating the increase of the MFI relative to control cells. AML subtypes are distinguished: filled symbols represent MO/M1/M2 AML subtypes whereas empty symbols represent M4/M5 subtypes. Mean values obtained from eight patients are represented.
**FIG. 4** depicts the change in c-Jun expression following treatment of HL60 cells with vitamin D3 (VD, 250nM), A24 (A24, 10µg/ml), deferoxamine (DFO, 5µM) or deferasirox (DFX, 3µM).

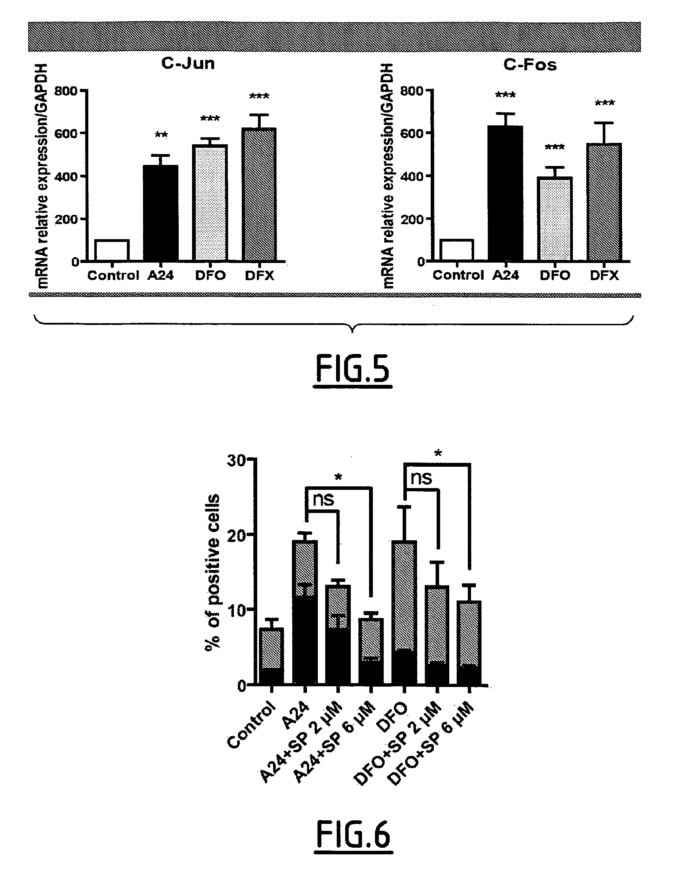

**FIG. 5** depicts the c-Fos and c-Jun mRNA levels (evaluated by quantitative RT-PCR and normalized to GAPDH mRNA levels) in HL60 cells treated for 48h with A24 (A24, 10µg/ml); deferoxamine (DFO, 5 µM); or deferasirox (DFX, 3 µM) (mean ± SEM, n=4).
**FIG. 6** depicts the early and late apoptosis/necrosis evaluated by flow cytometry using an annexin V-FITC (black) and annexin V-FITC/PI (grey) labeling. HL60 cells were cultured in the presence or in the absence of A24 (10 µg/ml) or deferoxamin (DFO, 5 µM) in a medium supplemented or not with JNK inhibitor SP600125 (SP) at 2µM and 6µM (mean ± SEM, n=3).

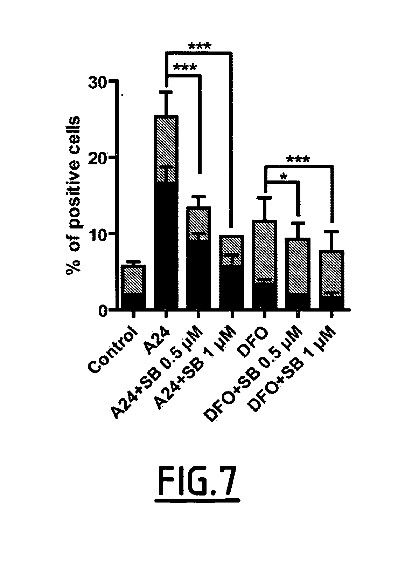

**FIG. 7** depicts the early and late apoptosis/necrosis evaluated by flow cytometry using an annexin V-FITC (black) and annexin V-FITC/PI (grey). HL60 cells were cultured in the presence or in the absence of A24 (A24, 10 µg/ml) or deferoxamin (DFO, 5 µM) in a medium supplemented or not with p38 inhibitor SB203580 (SB) at 0.5µM and 1 µM (mean ± SEM, n=3).

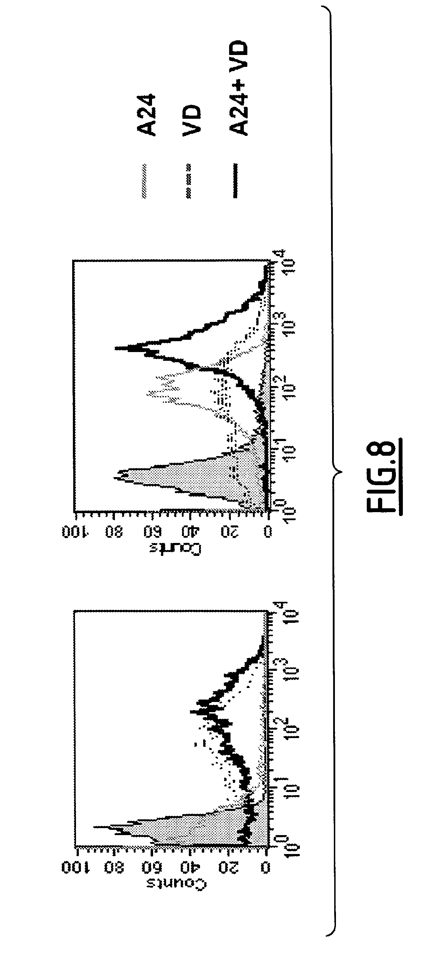

**FIG. 8** depicts the flow cytometry analysis of CD14 and CD11 b expression in HL60 cells treated for 72h with A24 (10 µg/ml) (continuous thin line), with VD (250 nM) (discontinuous thin line) or with the association of VD and iron-chelating agents (continuous bold line) Filled histograms represent control isotype labeling.

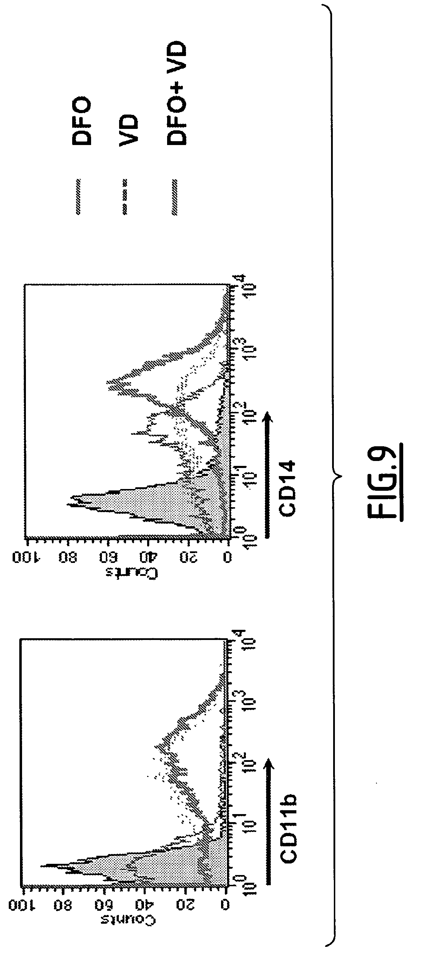

**FIG. 9** depicts the flow cytometry analysis of CD14 and CD11b expression in HL60 cells treated for 72h with DFO (5µM) (continuous thin line), with VD (250 nM) (discontinuous thin line) or with the association of VD and iron-chelating agents (continuous bold line) Filled histograms represent control isotype labeling.

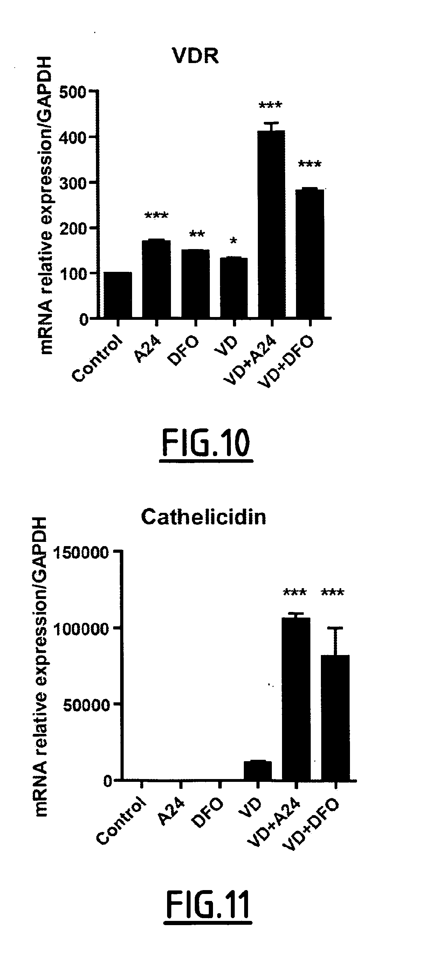

**FIG. 10** depicts the level of VDR transcript (evaluated by quantitative RT-PCR and normalized to GAPDH mRNA levels) in HL60 cells treated for 6h with A24 (A24/ 10 µg/ml); deferoxamin (DFO/ 5µM), or vitamin D3 (VD/250 nM), A24 and deferoxamin (A24+DFO/10 µg/ml and 5µM) deferoxamin and vitamin D3 (VD+DFO/ 250nM and 5µM).
**FIG. 11** depicts the level of cathelicidin transcript (evaluated by quantitative RT-PCR and normalized to GAPDH mRNA level) in HL60 cells treated for 6h with A24 (A24/ 10 µg/ml); deferoxamin (DFO/ 5µM), or vitamin D3 (VD/250 nM), A24 and deferoxamin (A24+DFO/10 µg/ml and 5µM), deferoxamin and vitamin D3 (VD+DFO/ 250nM and 5µM).

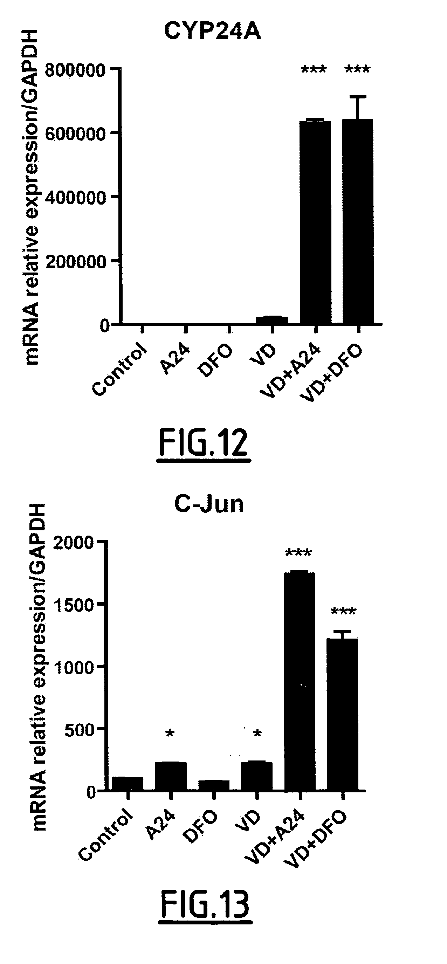

**FIG. 12** depicts the level of CYP24A transcript (evaluated by quantitative RT-PCR and normalized to GAPDH mRNA level) in HL60 cells treated for 6h with A24 (A24/ 10 µg/ml); deferoxamin (DFO/ 5µM), or vitamin D3 (VD/250 nM), A24 and deferoxamin (A24+DFO/10 µg/ml and 5µM), deferoxamin and vitamin D3 (VD+DFO/ 250nM and 5µM).
**FIG. 13** depicts the level of c-Jun transcript (evaluated by quantitative RT-PCR and normalized to GAPDH mRNA level) in HL60 cells treated for 16h with A24 (A24/ 10 µg/ml); deferoxamin (DFO/ 5µM), or vitamin D3 (VD/250 nM), A24 and deferoxamin (A24+DFO/10 µg/ml and 5µM), deferoxamin and vitamin D3 (VD+DFO/ 250nM and 5µM).

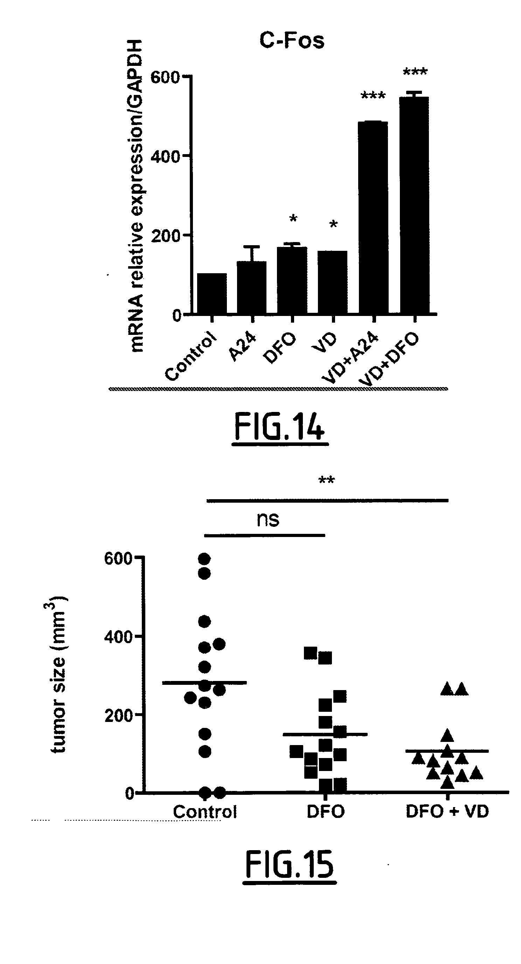

**FIG. 14** depicts the level of c-Fos transcript (evaluated by quantitative RT-PCR and normalized to GAPDH mRNA level) in HL60 cells treated for 16h with A24 (A24/ 10 µg/ml); deferoxamin (DFO/ 5µM), or vitamin D3 (VD/250 nM), A24 and deferoxamin (A24+DFO/10 µg/ml and 5µM), deferoxamin and vitamin D3 (VD+DFO/ 250nM and 5µM).
**FIG. 15** depicts the tumor sizes measured at day 25 in xenografted mice injected with deferoxamin (DFO, n=14) (DFO at 20 mg/day, 5-7 times/week), deferoxamin and vitamin D3 (VD at 1 µg, twice per week and DFO at 20 mg/day, 5-7 times/week) (DFO + VD, n=13) compared to mice injected with vehicle (control, n=12). Individual tumors are plotted.

### EXAMPLES

### METHODS

### Clinical samples and cell lines

Peripheral blood cells from AML patients and healthy donors was analyzed after obtaining their written informed consent (please refer to supplementary table 2 for additional information). Peripheral blood was collected at the initial diagnosis previously to treatment administration. Mononuclear cells were separated by Ficoll-hypaque (PAA laboratories) density centrifugation and resuspended in IMDM medium (Invitrogen) supplemented with 15% fetal calf serum (FCS) (Hyclone), 100 ng/ml Stem Cell Factor (SCF),10 ng/ml Interleukin 3 (IL-3) and 25 ng/ml FLT3-L (all purchased from R&D Systems). HL60 and U937 cell lines were a kind gift from J.M. Cayuela (Saint-Louis Hospital, Paris). Cells were cultured in RPMI-1640 medium (Invitrogen) supplemented with 5% FCS and antibiotics.

**Table 1: Characteristics of AML patients used in this study**

| Sexe | age | FAB subtype | WBC | (/mm3) % | blastes Karyotype |
|---|---|---|---|---|---|
| M | 42 | M4éo | 6400 | 21 | 46,XY,t(16;16)(p13;q22)[17]/46,XY [3] |
| M | 79 | M2/MDS | 5000 | 6 | 43,XY,-2,add(5)(q?31),-7,del(7)(q21),-13,-14,-16,-17,+3 mar [11] /44,idem,+8 [3] / 46,XY [4] |
| M | 53 | M1 | 4 200 | 70 | 47,XY,+21[5]/46,XY [15] |
| F | 49 | M5 | 160 000 | 77 | 46,XX [20] |
| M | 61 | M1 | 21 000 | 91 | 46,XY,del(9)(q12q33) [16] / 46,XY [4] |
| F | 23 | M5 | 200 000 | 98 | 46,XX,del(2)(q3?2),t(10;11)(p12;q23) |
| F | 59 | M4 | 60 0000 | 55 | 46,XX [20] |
| M | 36 | M2 | 6 0000 | 55 | failure |
| M | 51 | M5 | 165 000 | 80 | failure |
| M | 76 | M2 | 28 000 | 19 | 46,XY [20] |
| F | 68 | M2 | 112 000 | 90 | 46,XX [20] |
| F | 54 | M2 | 24 000 | 16 | 46,XX [20] |
| M | 40 | M2 | 9 300 | 43 | 45,X,-Y,t(8;21)(q22;q22) |
| M | 66 | M0 | 10 000 | 80 | 48,XY,?del(7)(q3?3q3?5),?del(20)(q1?2),+21,+21 |
| F | 64 | M1 | 27 400 | 85 | 81-92,XXXX [17] |

### Cell treatments and assays for proliferation

Deferasirox was provided by Novartis. VP16, DFO, VD and FeCl₃ were purchased from Sigma-Aldrich. mAb A24 was produced and purified as described (Moura et al. (2001) J Exp Med 194:417-25). Inhibitors of JNK (SP600125), ERK (PD98059) and p38 (SB203580) MAPK were all purchased from Santa-Cruz Biotechnology.

For proliferation assays, the HL60 cell line was resuspended in RPMI-1640 medium with 5% FCS, and patient blasts in IMDM medium with 15% BIT 9500 (Stem Cell Technologies). Cells were added in triplicate at 2.5x10⁴ per well in 96-well tissue culture plates (Falcon). Proliferation was measured as described in Lepelletier et al. ((2007) Cancer Res 67:1145-1154).

### Cytogenetic Analysis

Fluorescence in situ hybridization (FISH) was performed using standard protocols with specific centromeric probes for chromosome 8 (pZ8.4) and chromosome 12 (pBR12), using as control.

### RNA isolation, real-time quantitative PCR and transcriptome analysis

Total RNA was extracted using Nucleospin RNA II (Macherey-Nagel). Following DNAse treatement, first-strand cDNA was synthesized using a SuperScript II reverse transcriptase (Invitrogen). Quantitative real-time PCR was performed with a Chromo-IV PCR System (MJ Research) and PCR products were quantified using SybrGreen Technology (Jumpstart mastermix, Sigma Aldrich). Results of real-time quantitative PCR were interpreted using the delta-delta Ct method (Livak et al. (2001) Methods 25:402-408). The complete list of primer sequences used is available in supplementary data section, table 4.

For microarrays the Agilent 44 K Whole Human Genome (G4112A) long (60 bp) oligonucleotide microarray and the dual-color analysis method were used in which probes from treated samples and from reference RNA are differentially labeled with cyanine 5 and cyanine 3. These microarrays have 44.290 features with 41.000 distinct oligonucleotides belonging to 33.715 sequences defined by their accession number. cRNA from each treated sample was labeled with cyanine 3 (Cy3)-cytidine triphosphate (CTP) and the untreated HL60 RNA reference pool with cyanine 5 (Cy5)-CTP for direct comparison. Reverse transcription, linear amplification, cRNA labeling, and purification were performed with the Agilent linear amplification kit. Microarray data were mainly analyzed with Resolver software (Rosetta Inpharmatics). All data were filtered to eliminate low-intensity values under 50 arbitrary units for both colors.

### Tumor xenografts

For prevention of tumor establishment, 5.10⁶ HL60 cells mixed with Matrigel (1:1, V/V) and injected subcutaneously into 8-10 week-old female athymic nude mice. Mice were then injected i.p. with A24 (40 mg/kg, once at day 1), DFO (20 mg/day, 5 7times/week), VD (1 µg, twice a week) or PBS as a vehicle control during 25 days. Tumor growth was measured as described in Lepelletier *et al. ((2007) Cancer Res* **67**:1145-1154).

### Immunohistochemistry

Serial sections of 3 mm from the paraffin blocks were deparaffinized in xylene, and hydrated in a graded series of alcohol. Staining was performed using the Lab Vision Immunohistochemical Autostainer (Lab Vision Corporation, Fremont, CA) with primary antibodies against CD14, CD11b (Sigma-Aldrich, 1:200), p-ERK and p-Jun (Cell Signaling, 1:1000), followed by biotinylated anti-Ig and streptavidin-peroxidase, and developed in PBS with DAB (0.05 %) and 0.003 % H₂O₂. Images were obtained using IM50 software on a digital camera (DFC320, Leica) and a Leica microscope (DM-2000).

### Statistical analysis

Data are expressed as means ± SEM. Statistical analysis were performed with GraphPadPrism 5 software. Student's t-tests was used to compare two groups, whereas multigroup comparisons were made using one-way ANOVA test followed by post-hoc analysis (Bonferroni test). Kruskal-Wallis test followed by Dunn's post-test were used for non-parametric comparisons where indicated. For comparison of tumor-free animal curves, Log-rank test were used. Results were considered statistically significant at a P value less than 0.05 (*), less than 0.01 (**) or less than 0.001 (***).

### RESULTS

### Example 1: Iron deprivation affects the expression of gene related with cell differentiation

Molecular mechanisms implicated in the anti-tumor effect of the aforementioned iron-chelating therapeutic approaches remain to be fully elucidated. To gain new insights into these mechanisms genes were identified that are affected both by iron chelators (deferoxamine (DFO) and deferasirox (DFX)) and by TfR1 impairment (mAb A24). The rationale was that following this strategy would decrease the probability to select irrelevant genes. These studies focused in a cell line model (HL60) to avoid heterogeneity in data related to the several genetic events implicated in patients AML oncogenesis. Analysis of the collected data revealed that 105 genes were modulated by the three different agents. Among these genes, several were related to cell differentiation. In particular, markers of monocytes or activated macrophages were up-regulated (SERPINB8, ITGB2, TREML2, ITGAM) whereas those of granulocyte differentiation were down-regulated (ELA2, PRTN3, CLC, AZU) by iron-deprivation agents.

### Example 2: Iron homeostasis plays a role in granulo-monocytic differentiation

The effect of iron deprivation in primary hematopoietic progenitors was tested. Semisolid cultures of primary CD34⁺ cells (with pluripotent cytokines) in the presence or absence of iron deprivation agents (directly as does iron chelators or indirectly by the use of the anti-TfR mAb A24) led to increased number of CFU-M colonies to the detriment of CFU-G suggesting that chelating iron from hematopoietic precursors induced commitment towards monocytic over granulocytic lineage (**FIG. 1**) although it did not change total numbers of colonies. In liquid cultures, iron deprivation did not affect cord blood cells proliferation but modulated their commitment towards monocytic lineage. Likewise, there was a consistent up-regulation of transcription factors specific of the monocytic lineage HOXA10, EGR1 and MafB. Cytological evaluation of cultures confirmed that monocytic cells numbers were enhanced in iron-poor cultures. Thus, in bone marrow niches, iron availability could be a factor determining the myelo-monocytic versus granulocytic outcome of hematopoietic progenitors differentiation.

### Example 3: Iron deprivation induce leukemia cells differentiation

The ability of iron deprivation to override blockade of AML cells differentiation was tested. In AML cells lines, monocytic markers CD14 and CD11b (which are induced by the vitamin D3 (VD), a monocytic differentiating agent) were induced by iron deprivation by A24 mAb, DFO and DFX. Induction of cell surface markers was accompanied by characteristic cytological modifications of monocytes. Iron deprivation induced cytoplasm enlargement, loss of cytoplasm basophilia and azurophilic granules. Up-regulation of differentiation markers expression was abrogated when cultures where supplemented with an excess of soluble iron confirming that the primary origin of the monocytic differentiation program is dependent on iron availability to cells.

Next, it was investigated whether cells from AML patients could also be induced to differentiate upon iron deprivation. Fresh AML blasts from different AML subtypes were isolated at the time of diagnosis (the complete list of subtypes and biological parameters of patients used in the study are listed in **Table 1**) and cultured in the presence of mAb A24 and DFO. CD14 and CD11b markers expression were consistently up-regulated in cultures indicating that blasts from different AML subtypes, even if heterogeneous in their oncogenic events leading to differentiation arrest, are susceptible to differentiation therapy based on iron deprivation (**FIG. 2** and **FIG. 3**). *In vitro* dose-range studies showed that differentiation was accompanied by cell proliferation arrest and apoptosis.

### Example 4: JUN kinase pathways induced by iron deprivation

By comparing (with a non-supervised analysis), the pattern of genes induced by VD and iron-chelating agents, a high similarity was found among modulated genes. Thirty of the 105 genes modulated by iron deprivation were shared with VD modified genes. This finding underscores the potential similarities between both differentiation approaches. Both VD and iron-chelating agents up-regulated markers of monocytes (SERPINB8, ITGB2, TREML2, ITGAM) and down-regulated neutrophils markers (ELA2, PRTN3, CLC, AZU). JUN kinase pathway (JNK) is a major MAPK pathway implicated in monocytic differentiation and has been previously shown to be induced by VD (Wang et al; (2003) J Cell Biochem 89:1087-1101). Effectively, c-Jun gene was induced by VD but also by iron deprivation (**FIG. 4**). Moreover, transcripts coding for c-Fos and c-Jun were also up-regulated by both treatments (**FIG. 5**). Inhibition of the JNK by its specific inhibitor SP600125 abrogated differentiation induced by iron with A24 or DFO. MAPK pathways (extracellular signal-regulated kinase-ERK, JNK and p38) frequently converge to amplify signal transduction (Johnson et al. (2002) Science 298:1911-1912, Lopez-Bregami et al. (2007) Cancer Cell 11:447-460). In agreement, it was found that iron-chelating agents induced activation of the MAPK-related ERK and p38 pathways as evidence by the phosphorylation of their molecular effectors. Moreover, selective inhibition of ERK and p38 pathways blocked cell differentiation induced by iron deprivation. However, inhibitors of JNK and p38 (**FIG. 6** **and** **FIG. 7**) but not of ERK prevented cell apoptosis induced by iron deprivation.

### Example 5: Association of iron-chelating and vitamin D3 has a synergistic effect on AML blast differentiation

Next, it was investigated whether the association of iron-chelating agents and VD could have a synergistic effect. Association of chelators with VD drastically increased up-regulation of monocytic cell differentiation markers (**FIG. 8** and **FIG. 9**). This could be explained by the up-regulation of VDR gene transcription (**FIG. 10**) which has been implicated in monocytic cell differentiation (Himes et al; (2006) J Immunol 176:2219-2228, Wang et al. (2003) J Cell Biochem 89:1087-1101). The physiological relevance of the increased VDR gene expression was evidenced by the robust increase in the transcription of its downstream target genes, cathelicidin (1000 fold increase) (**FIG. 11**) and CYP24A (6000 fold increase) (**FIG. 12**). The synergy observed is also due to the activation of the JNK pathway as the c-Jun and c-Fos genes were notably up-regulated by iron-chelation and VD association (**FIG. 13** and **FIG. 14**). Moreover, phosphorylation of JNK increased when VD is associated to iron deprivation. These profound modifications were accompanied by morphological changes in treated cells. Therefore, association of both iron deprivation and differentiating agents such as VD, could reduce side-effects in combined therapy while keeping their anti-leukemic properties.

### Example 6: Association of iron-chelating and vitamin D3 has a synergistic effect in vivo in a mouse model of tumor xenografts

The efficacy of the differentiation therapy was further evaluated *in vivo* in a mouse model of tumor xenografts. Combination of VD with DFO therapy significantly reduced tumor growth in mice (**FIG. 15**). Reduced tumor growth was associated with apoptosis of tumor cell, features of cell differentiation and MAPK phosphorylation (ERK and JNK).

### Example 7: Association of iron-chelating and vitamin D3 induced blast differentiation in one AML patient

The effectiveness of iron chelator and VD was evaluated in one AML patient. A 69-year-old man had a recent transformation of myelodysplastic syndrome (MDS) into acute myeloid leukemia. Following high dose chemotherapy the patient remained in no blastic aplasia. Seven months after the diagnosis, blasts reappeared in the blood and treatment with iron chelator (deferasirox 1 g/day) and VD (4000UI/day) was initiated. This combined therapy was associated with an increase of neutrophils number and did not induced hypercalcemia. Importantly, combined treatment induced blast differentiation towards monocytic lineage as observed on MGG blood smears. This observation was further confirmed by cell sorting followed by cytogenetic analysis where the original blast trisomy was found in mature monocytes derived from immature cells but not in lymphocytes and NK cells. Therefore, iron homeostasis plays a key role in the control of cell differentiation in both normal and pathological situations. In iron-poor environments BM cells are prone to monocytic differentiation and blasts from AML could undergo differentiation in the presence of iron chelators. Genome screening shows that genes associated with cell differentiation are targeted by iron deprivation and are highly shared (30% of similarity) with VD.

## Claims

1. A pharmaceutical composition comprising:
a) at least one iron uptake inhibitor selected from the group consisting of an iron chelator and a transferrin receptor inhibitor, and
b) at least one vitamin D and/or at least one analog thereof and/or at least one vitamin D receptor modulator,
and optionally a pharmaceutically acceptable carrier.

2. The pharmaceutical composition according to claim 1, wherein the iron chelator is selected from the group consisting of deferoxamine, deferasirox, deferiprone, Triapine and di-2-pyridylketone-4,4,-dimethyl-3-thiosemicarbazone (Dp44mT).

3. The pharmaceutical composition according to claim 1, wherein the transferrin receptor inhibitor is an anti-transferrin receptor antibody

4. The pharmaceutical composition according to claim 3, wherein the anti-transferrin receptor antibody is monoclonal antibody A24 deposited at the CNCM (Collection nationale de Cultures de Microorganismes, 25 rue du Docteur Roux, Paris) on May 10, 2001, under number I-2665.

5. The pharmaceutical composition any one of claims 1 to 4, wherein the at least one vitamin D is vitamin D3.

6. The pharmaceutical composition according to any one of claims 1 to 5, wherein the at least one analog of vitamin D is selected from the group consisting of cholecalciferol, paricalcitriol, Maxacalcitol, 22-oxa-1α,25(OH)2D3 (OCT) and, 1α-dihydroxy-22,24-diene-24,26,27-trihomo-vitamin D3 (EB1089).

7. The pharmaceutical composition according to any one of claims 1 to 6, wherein the at least one vitamin D receptor modulator is selected from the group consisting of LY2108491, LY2109866 and LG190119.

8. The pharmaceutical composition according to any one of claims 1 to 7, comprising:
- vitamin D3, and
- monoclonal antibody A24 deposited at the CNCM (Collection nationale de Cultures de Microorganismes, 25 rue du Docteur Roux, Paris) on May 10, 2001, under number I-2665.

9. The pharmaceutical composition according to any one of claims 1 to 7, comprising:
- vitamin D3, and
- deferoxamine and/or deferasirox.

10. The pharmaceutical composition according to any one of claims 1 to 9, for use in the prevention or treatment of cancer.

11. The pharmaceutical composition for use according to claim 10, wherein the cancer is a hematopoietic malignancy.

12. The pharmaceutical composition for use according to claim 10 or 11, wherein the cancer is selected from the group consisting of myelodysplastic syndrome and acute myeloid leukemia.

13. At least one iron uptake inhibitor as defined in claims 1 to 4, and at least one vitamin D and/or a least one analog thereof and/or at least one vitamin D receptor modulator as defined in claims 5, 6 and 7 for use in the prevention or the treatment of cancer as defined in claims 10 to 12.

14. Products containing:
a) at least one iron uptake inhibitor as defined in claims 1 to 4, and
b) at least one vitamin D and/or at least one analog thereof and/or at least one vitamin D receptor modulator as defined in claims 5, 6 and 7,
as a combined preparation for simultaneous, separate or sequential use in the prevention or treatment of cancer as defined in claims 10 to 12.

## Patentansprüche

1. Eine pharmazeutische Zusammensetzung umfassend:
a) mindestens einen Eisenaufnahme-Inhibitor ausgesucht aus der Gruppe bestehend aus einem Eisen-Chelator und einem Transferrinrezeptor-Inhibitor und
b) mindestens ein Vitamin D und/oder mindestens ein Analog davon und/oder mindestens ein Vitamin D Rezeptor Modulator,
und optional ein pharmazeutisch verträglicher Träger.

2. Die pharmazeutische Zusammensetzung nach Anspruch 1, wobei der Eisen-Chelator aus der Gruppe bestehend aus Deferoxamin, Deferasirox, Deferipron, Triapin und di-2-Pyridylketon-4,4,-dimethyl-3-thiosemicarbazon (Dp44mT) ausgewählt ist.

3. Die pharmazeutische Zusammensetzung nach Anspruch 1, wobei der Transferrinrezeptor-Inhibitor ein Anti-Transferrinrezeptor Antikörper ist.

4. Die pharmazeutische Zusammensetzung nach Anspruch 3, wobei der Anti-Transferrinrezeptor Antikörper der monoklonale Antikörper A24, der im CNCM (Collection nationale de Cultures de Microorganismes, 25 rue du Docteur Roux, Paris) am 10. Mai 2001 unter der Nummer I-2665 hinterlegt wurde, ist.

5. Die pharmazeutische Zusammensetzung nach einem der Ansprüche 1 bis 4, wobei das mindestens eine Vitamin D Vitamin D3 ist.

6. Die pharmazeutische Zusammensetzung nach einem der Ansprüche 1 bis 5, wobei das mindestens eine Vitamin D Analog aus der Gruppe bestehend aus Cholecalciferol, Paricalcitriol, Maxacalcitol, 22-oxa-1α,25(OH)2D3 (OCT) und 1α-dihydroxy-22,24-diene-24,26,27-trihomo-Vitamin D3 (EB1089) ausgewählt ist.

7. Die pharmazeutische Zusammensetzung nach einem der Ansprüche 1 bis 6, wobei der mindestens eine Vitamin D Rezeptor Modulator aus der Gruppe bestehend aus LY2108491, LY2109866 und LG190119 ausgewählt ist.

8. Die pharmazeutische Zusammensetzung nach einem der Ansprüche 1 bis 7, umfassend:
- Vitamin D3 und
- den monoklonalen Antikörper A24, der im CNCM (Collection nationale de Cultures de Microorganismes, 25 rue du Docteur Roux, Paris) am 10. Mai 2001 unter der Nummer 1-2665 hinterlegt wurde.

9. Die pharmazeutische Zusammensetzung nach einem der Ansprüche 1 bis 7, umfassend:
- Vitamin D3 und
- Deferoxamin und/oder Deferasirox.

10. Die pharmazeutische Zusammensetzung nach einem der Ansprüche 1 bis 9 zur Verwendung in der Prävention oder Behandlung von Krebs.

11. Die pharmazeutische Zusammensetzung zur Verwendung nach Anspruch 10, wobei der Krebs eine hämatopoetische maligne Erkrankung ist.

12. Die pharmazeutische Zusammensetzung zur Verwendung nach Anspruch 10 oder 11, wobei der Krebs aus der Gruppe bestehend aus myelodysplastischem Syndrom und akuter myeloischer Leukämie ausgewählt ist.

13. Mindestens einen Eisenaufnahme-Inhibitor wie in den Ansprüchen 1 bis 4 definiert und mindestens ein Vitamin D und/oder mindestens ein Analog davon und/oder mindestens ein Vitamin D Rezeptor Modulator wie in den Ansprüchen 5, 6 und 7 definiert zur Verwendung in der Prävention oder Behandlung von Krebs wie in den Ansprüchen 10 bis 12 definiert.

14. Produkte beinhaltend:
a) mindestens einen Eisenaufnahme-Inhibitor wie in den Ansprüchen 1 bis 4 definiert und
b) mindestens ein Vitamin D und/oder mindestens ein Analog davon und/oder mindestens ein Vitamin D Rezeptor Modulator wie in den Ansprüchen 5, 6 und 7 definiert als kombiniertes Präparat zur simultanen, separaten oder sequenziellen Verwendung in der Prävention oder Behandlung von Krebs wie in den Ansprüchen 10 bis 12 definiert.

## Revendications

1. Composition pharmaceutique comprenant :
a) au moins un inhibiteur de l'absorption du fer sélectionné dans le groupe consistant en un chélateur du fer et un inhibiteur du récepteur de la transferrine, et
b) au moins une vitamine D et/ou au moins un analogue de celle-ci et/ou au moins un modulateur du récepteur de la vitamine D,
et optionnellement un véhicule pharmaceutiquement acceptable.

2. Composition pharmaceutique selon la revendication 1, dans laquelle le chélateur du fer est sélectionné dans le groupe consistant en la déféroxamine, le déférasirox, la défériprone, la triapine et la di-2-pyridylcétone-4,4-diméthyl-3-thiosemicarbazone (Dp44mT).

3. Composition pharmaceutique selon la revendication 1, dans laquelle l'inhibiteur du récepteur de la transferrine est un anticorps anti-récepteur de la transferrine.

4. Composition pharmaceutique selon la revendication 3, dans laquelle l'anticorps anti-récepteur de la transferrine est l'anticorps monoclonal A24 déposé auprès de la CNCM (Collection nationale de Cultures de Microorganismes, 25 rue du Docteur Roux, Paris) le 10 mai 2001 sous le numéro I-2665.

5. Composition pharmaceutique selon l'une quelconque des revendications 1 à 4, dans laquelle la au moins une vitamine D est la vitamine D3.

6. Composition pharmaceutique selon l'une quelconque des revendications 1 à 5, dans laquelle le au moins un analogue de vitamine D est sélectionné dans le groupe consistant en le cholécalciférol, le paricalcitriol, le maxacalcitol, la 22-oxa-1α,25(OH)2D3 (OCT) et la 1α-dihydroxy-22,24-diène-24,26,27-trihomo-vitamine D3 (EB1089).

7. Composition pharmaceutique selon l'une quelconque des revendications 1 à 6, dans laquelle le au moins un modulateur du récepteur de la vitamine D est sélectionné dans le groupe consistant en LY2108491, LY2109866 et LG190119.

8. Composition pharmaceutique selon l'une quelconque des revendications 1 à 7, comprenant :
- de la vitamine D3, et
- l'anticorps monoclonal A24 déposé auprès de la CNCM (Collection nationale de Cultures de Microorganismes, 25 rue du Docteur Roux, Paris) le 10 mai 2001 sous le numéro I-2665.

9. Composition pharmaceutique selon l'une quelconque des revendications 1 à 7, comprenant :
- de la vitamine D3, et
- de la déféroxamine et/ou du déférasirox.

10. Composition pharmaceutique selon l'une quelconque des revendications 1 à 9, pour son utilisation dans la prévention ou le traitement du cancer.

11. Composition pharmaceutique pour son utilisation selon la revendication 10, dans laquelle le cancer est une malignité hématopoïétique.

12. Composition pharmaceutique pour son utilisation selon la revendication 10 ou 11, dans laquelle le cancer est sélectionné dans le groupe consistant en un syndrome myélodysplasique et la leucémie myéloïde aiguë.

13. Au moins un inhibiteur de l'absorption du fer tel que défini dans les revendications 1 à 4, et au moins une vitamine D et/ou au moins un analogue de celle-ci et/ou au moins un modulateur du récepteur de la vitamine D tels que définis dans les revendications 5, 6 et 7, pour leur utilisation dans la prévention ou le traitement du cancer tel que défini dans les revendications 10 à 12.

14. Produits contenant :
a) au moins un inhibiteur de l'absorption du fer tel que défini dans les revendications 1 à 4, et
b) au moins une vitamine D et/ou au moins un analogue de celle-ci et/ou au moins un modulateur du récepteur de la vitamine D tels que définis dans les revendications 5, 6 et 7,
en tant que préparation combinée pour une utilisation simultanée, séparée ou séquentielle dans la prévention ou le traitement du cancer tel que défini dans les revendications 10 à 12.
